# EUROPEAN PATENT APPLICATION

(11) **EP 4 596 533 A1**
(43) Date of publication of application: **06.08.2025**
(21) Application number: 23871464.6
(22) Date of filing: 03.08.2023
(51) Int. Cl.: C07C 269/04, C01G 25/02, C07D 233/34

(54) **CARBON DIOXIDE SUPPLY CONVERSION AGENT AND CARBON DIOXIDE UTILIZATION METHOD**

(30) Priority: 30.09.2022 WO PCT/JP2022/036820
(71) Applicant: Tohoku University, Sendai-shi, Miyagi 980-8577 (JP)
(72) Inventor: YOSHIOKA, Toshiaki, Sendai-shi, Miyagi 980-8577 (JP); KAMEDA, Tomohito, Sendai-shi, Miyagi 980-8577 (JP); TOMISHIGE, Keiichi, Sendai-shi, Miyagi 980-8577 (JP); FUKUSHIMA, Yasuhiro, Sendai-shi, Miyagi 980-8577 (JP); YABUSHITA, Mizuho, Sendai-shi, Miyagi 980-8577 (JP); HIROMORI, Kousuke, Sendai-shi, Miyagi 980-8577 (JP); SUZUKI, Ukyo, Sendai-shi, Miyagi 980-8577 (JP)
(74) Representative: Cabinet Becker et Associés
(86) International application number: PCT/JP2023/028442
(87) International publication number: WO 2024/070222

(57) **Abstract**

A carbon dioxide supply conversion agent of the present invention is a carbon dioxide supply conversion agent to be used for a reaction with an alkylamine compound. The carbon dioxide supply conversion agent contains: an oxide; and carbon dioxide adsorbed on a surface of the oxide.

## Description

### Technical Field

The present invention relates to a carbon dioxide supply conversion agent and a carbon dioxide utilization method.

The present application is based on PCT/JP2022/036820 filed on September 30, 2022, and the contents thereof are incorporated herein.

### Background Art

Greenhouse gas emissions, which are believed to be one of causes of global warming, have become a global issue, and countries around the world are making efforts to reduce emissions of carbon dioxide (CO₂), which accounts for 90.8% of greenhouse gases.

One approach to reduce a net CO₂ emission amount is to directly collect exhaust gas or CO₂ from the atmosphere without using a plant. Examples of a destination of CO₂ thus collected include sequestration underground or on the ocean floor, or effective utilization by reacting CO₂ with other substances to synthesize useful chemicals. As a specific method for effectively utilizing CO₂, for example, there has been known a method of reacting CO₂ with hydrogen to synthesize hydrocarbons such as methane. However, the method utilizes a reduction reaction and requires a large amount of energy.

On the other hand, there also has been known a method of effectively utilizing CO₂ through a non-reductive reaction, such as synthesizing ethylene urea from ethylenediamine and CO₂ (Non Patent Literatures 1 to 5). In Non Patent Literature 1, ethylene urea is synthesized by reacting ethylenediamine with high-pressure CO₂ (0.5 MPa) at 140°C to 160°C in the presence of a solid catalyst. As described above, in the method, high-pressure CO₂, that is, a large excess CO₂ is required, and CO₂ utilization efficiency is low. In Non Patent Literatures 2 to 5, first, CO₂ is blown into an amine (such as ethylenediamine) to chemically absorb the CO₂ and produce a corresponding carbamic acid. The produced carbamic acid is used as a reaction substrate and reacted with a catalyst at an appropriate reaction temperature to obtain desired products, such as ethylene urea and carbamic acid esters. In the method, high-pressure CO₂ is not required. On the other hand, CO₂ is supplied to a reaction system by being blown in a state of gas from the air. Since a proportion of CO₂ in the air is about 0.04%, it is necessary to blow a large amount of air in order to sufficiently supply CO₂. However, when a large amount of air is blown into the reaction system, since a solvent and ethylenediamine are vaporized in an amount equivalent to a saturated vapor pressure of the air, the reaction efficiency decreases, and as a result, the utilization efficiency of carbon dioxide decreases.

### Citation List

### Non Patent Literature

Non Patent Literature 1: M. Tamura, K. Noro, M. Honda, Y. Nakagawa, K. Tomishige, Green Chem., 15, 1567-1577(2013).
Non Patent Literature 2: H. Koizumi, K. Takeuchi, K. Matsumoto, N. Fukaya, K. Sato, M. Uchida, S. Matsumoto, S. Hamura, J.-C. Choi, Commun. Chem., 4, 66(2021).
Non Patent Literature 3: H. Koizumi, K. Takeuchi, K. Matsumoto, N. Fukaya, K. Sato, M. Uchida, S. Matsumoto, S. Hamura, J.-C. Choi, ACS Sustainable Chem. Eng., 10, 5507-5516(2022).
Non Patent Literature 4: J. Peng, M. Tamura, M. Yabushita, R. Fujii, Y. Nakagawa, K. Tomishige, ACS Omega, 6, 27527-27535(2021).
Non Patent Literature 5: J. Peng, M. Yabushita, Y. Li, R. Fujii, M. Tamura, Y. Nakagawa, K. Tomishige, Appl. Catal. A: Gen., 643, 118747(2022).

### Summary of Invention

### Technical Problem

The present invention has been made in view of the above circumstances, and an object of the invention is to provide a carbon dioxide supply conversion agent to be reacted with an alkylamine compound, and to provide a carbon dioxide utilization method that can increase utilization efficiency of carbon dioxide by reacting the carbon dioxide with an alkylamine compound.

### Solution to Problem

In order to solve the above problems, the present invention employs the following methods.
(1) A carbon dioxide supply conversion agent according to one aspect of the present invention is a carbon dioxide supply conversion agent to be used for a reaction with an alkylamine compound, and the carbon dioxide supply conversion agent contains: an oxide; and carbon dioxide adsorbed on a surface of the oxide.
(2) In the carbon dioxide supply conversion agent according to the above (1), the oxide may be a titanium zirconium oxide represented by a compositional formula TiₓZr₍₁₋ₓ₎O₂, where 0 < x < 1.
(3) In the carbon dioxide supply conversion agent according to the above 1, the oxide may be a titanium zirconium oxide represented by a composition formula TiₓZr_{y}Ce_{z}O₂, where 0 < x < 1,0 < y < 1, and 0 < z < 1.
(4) In the carbon dioxide supply conversion agent according to the above (1), the oxide may be TiO₂.
(5) In the carbon dioxide supply conversion agent according to the above (1), the oxide may be ZrO₂.
(6) In the carbon dioxide supply conversion agent according to the above (1), the oxide may be CeO₂.
(7) In the carbon dioxide supply conversion agent according to the above (1), the oxide may be a Mg-Al-based layered double hydroxide containing Mg and Al as a constituent metal, and a ratio (Mg/Al) of the number of moles of the Mg contained to the number of moles of the Al contained may be 2 or more and 4 or less.
(8) In the carbon dioxide supply conversion agent according to the above (7), the Mg-Al-based layered double hydroxide may be doped with Ce.
(9) In the carbon dioxide supply conversion agent according to the above (7), the Mg-Al-based layered double hydroxide may be doped with Zr.
(10) In the carbon dioxide supply conversion agent according to the above (1), the oxide may be Ce(OH)₄.
(11) In the carbon dioxide supply conversion agent according to the above (1), the oxide may be Zr(OH)₄.
(12) A carbon dioxide utilization method according to one aspect of the present invention is a carbon dioxide utilization method by producing an alkylurea compound using the carbon dioxide supply conversion agent according to the above (1) to (11), and the carbon dioxide utilization method includes: reacting the carbon dioxide adsorbed on a surface of the oxide with an alkylamine compound represented by the following general formula (1) in the presence of a metal oxide catalyst to produce an alkylurea compound represented by the following general formula (2).
   (In the general formula (1) and the general formula (2), R¹ and R² each represent a hydrogen atom, an alkyl group having 1 to 6 carbon atoms, a benzyl group, an aminoalkyl group having 1 to 6 carbon atoms, or a hydroxyalkyl group having 1 to 6 carbon atoms. A combination of R¹ and R² may be any combination except for a combination in which both R¹ and R² are a hydrogen atom. R¹ in the general formula (1) and R¹ in the general formula (2) are the same, and the same applies to R².) 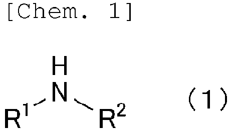 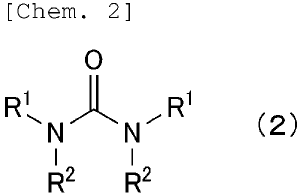
(13) In the carbon dioxide utilization method according to the above (12), it is preferable that the carbon dioxide and the alkylamine compound are reacted in a pressurized state of 0.1 MPa or more and 1.0 MPa or less.
(14) In the carbon dioxide utilization method according to the above (12), it is preferable that the carbon dioxide and the alkylamine compound are reacted in a heated state of 0°C or higher and 200°C or lower.

### Advantageous Effects of Invention

The present invention provides a carbon dioxide supply conversion agent, therefore carbon dioxide can be reacted with an alkylamine compound. The present invention also provides a carbon dioxide utilization method that can increase utilization efficiency of carbon dioxide by reacting the carbon dioxide with an alkylamine compound.

### Brief Description of Drawings

[FIG. 1] FIG. 1 is a configuration diagram of a gas adsorption device used in a carbon dioxide utilization method of the present invention.
[FIG. 2] FIG. 2 is a cross-sectional view of an autoclave used in the carbon dioxide utilization method of the present invention.
[FIG. 3] FIG. 3 is a graph illustrating a result of X-ray diffraction measurement of titanium zirconium oxide and cerium oxide in Example 1.
[FIG. 4] FIG. 4 illustrates adsorption isotherms obtained for the titanium zirconium oxide and cerium oxide in Example 1.
[FIG. 5] FIG. 5 is a graph in which produced amounts of ethylene urea and 2-piperazinone by a reaction between ethylenediamine (EDA) and the titanium zirconium oxide and cerium oxide in Example 1 are compared.
[FIG. 6] FIG. 6 illustrates an adsorption-desorption isotherm obtained for titanium zirconium oxide in Example 2.
[FIG. 7] FIG. 7 is a total ion chromatogram obtained for titanium zirconium oxide in Example 3.
[FIG. 8] FIG. 8 illustrates an adsorption isotherm obtained for titanium zirconium oxide in Example 4.
[FIG. 9] FIG. 9 illustrates an adsorption-desorption isotherm obtained for titanium zirconium oxide in Example 5.
[FIG. 10] FIG. 10 is a total ion chromatogram obtained for titanium zirconium oxide in Example 6.
[FIG. 11A] FIG. 11A is a graph illustrating a result of X-ray diffraction measurement of a Ce-doped Mg-Al-based layered double hydroxide in Example 7.
[FIG. 11B] FIG. 11B is a graph illustrating a result of X-ray diffraction measurement of a Ce-doped Mg-Al-based layered double hydroxide in Example 8.
[FIG. 12A] FIG. 12A is an SEM image of the Ce-doped Mg-Al-based layered double hydroxide in Example 7.
[FIG. 12B] FIG. 12B is an SEM image of a Ce-doped Mg-Al-based layered double hydroxide in Example 8.
[FIG. 13A] FIG. 13A is elemental mapping of the Ce-doped Mg-Al-based layered double hydroxide in Example 7.
[FIG. 13B] FIG. 13B is elemental mapping of the Ce-doped Mg-Al-based layered double hydroxide in Example 8.
[FIG. 14A] FIG. 14A is a graph illustrating a result of carbon dioxide gas adsorption measurement by the Ce-doped Mg-Al-based layered double hydroxide in Example 7.
[FIG. 14B] FIG. 14B is a graph illustrating a result of carbon dioxide gas adsorption measurement by the Ce-doped Mg-Al-based layered double hydroxide in Example 8.
[FIG. 15] FIG. 15 is a diagram illustrating a process flow for oxide synthesis in Example 11.
[FIG. 16] FIG. 16 is a graph illustrating an X-ray diffraction pattern of Ce(OH)₄ synthesized in Example 11.
[FIG. 17] FIG. 17 is a graph illustrating a CO₂-TPD profile of Ce(OH)₄ obtained in Example 12.
[FIG. 18A] FIG. 18A is a graph illustrating a relationship between a reaction temperature during synthesis of ethylene urea and a produced amount of ethylene urea in Examples 13 to 17.
[FIG. 18B] FIG. 18B is a graph illustrating a relationship between a reaction temperature during synthesis of 2-piperazinone and a produced amount of 2-piperazinone in Examples 13 to 17.

### Description of Embodiments

Hereinafter, a carbon dioxide supply conversion agent and a carbon dioxide utilization method according to embodiments to which the present invention is applied will be described in detail with reference to the drawings. The drawings used in the following description may show feature portions in an enlarged manner for convenience in order to make features easier to be understood, and dimensional ratios of components may not be the same as in reality. A material, a dimension, and the like exemplified in the following description are merely examples, and the invention is not limited thereto, and can be appropriately modified and implemented without departing from the gist of the present invention.

### <First Embodiment>

### [Carbon Dioxide Supply Conversion Agent]

A carbon dioxide supply conversion agent according to a first embodiment of the present invention is a carbon dioxide supply conversion agent to be used for a reaction with an alkylamine compound, and mainly includes an oxide and carbon dioxide adsorbed on a surface of the oxide. The carbon dioxide supply conversion agent enables adsorption and desorption of carbon dioxide during the reaction with an alkylamine compound, functions as a supply agent that supplies carbon dioxide, and also functions as a conversion agent that promotes conversion of the alkylamine compound into a useful compound.

The oxide may be any oxide capable of adsorbing carbon dioxide on a surface thereof. For example, a specific surface area is preferably 10 m²/g or more and 700 m²/g or less, and more preferably 50 m²/g or more and 500 m²/g or less.

As such an oxide, a titanium zirconium oxide represented by a compositional formula TiₓZr₍₁₋ₓ₎O₂, where 0 < x < 1, or TiO₂, ZrO₂, and CeO₂ can be used. In the present embodiment, the titanium zirconium oxide is used as an example. The titanium zirconium oxide has a basic site derived from ZrO₂ on the surface, and can adsorb carbon dioxide to the basic site through acid-base interaction. The carbon dioxide adsorbed to the basic site is desorbed at a high temperature of 200°C to 800°C. The carbon dioxide is also physically adsorbed to a portion having no basic site in a micropore, but in this case, since an adsorption force is weak, the carbon dioxide is desorbed at a low temperature of 50°C to 200°C.

### [Method for Producing Alkylurea Compound]

A carbon dioxide utilization method of the present invention is a carbon dioxide utilization method by producing (synthesizing) an alkylurea compound, which is a useful compound, using the carbon dioxide supply conversion agent described above. Specifically, in the presence of a metal oxide catalyst, carbon dioxide adsorbed on the surface of the oxide is reacted with an alkylamine compound represented by the following general formula (1) to produce an alkylurea compound represented by the following general formula (2).

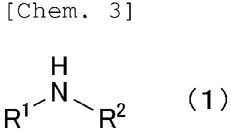

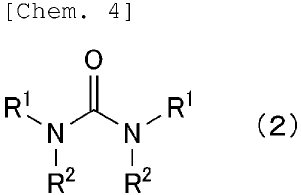

In the general formula (1) and the general formula (2), R¹ and R² each represent a hydrogen atom, an alkyl group having 1 to 6 carbon atoms, a benzyl group, an aminoalkyl group having 1 to 6 carbon atoms, or a hydroxyalkyl group having 1 to 6 carbon atoms. A combination of R¹ and R² may be any combination except for a combination in which both R¹ and R² are a hydrogen atom. R¹ in the general formula (1) and R¹ in the general formula (2) are the same, and the same applies to R².

When titanium zirconium oxide is used as the oxide, an alkylurea compound is produced mainly through the following three steps (first step, second step, and third step).

### (First Step)

Titanium zirconium oxide (Ti-Zr-based oxide) is synthesized, which is composed of a composition represented by a composition formula TiₓZr₍₁₋ₓ₎O₂, where 0 < x < 1, and has a performance as an adsorbent for carbon dioxide. The titanium zirconium oxide can be synthesized by, for example, a combination of a sol-gel method and a solvothermal method.

In the sol-gel method, a mixed solution of ethanol and potassium chloride is used as a solvent, hexadecylamine is added thereto, and titanium (IV) isopropoxide (TIP) and zirconium (IV) propoxide (ZrP) are used as a raw material of TiₓZr₍₁₋ₓ₎O₂. TIP and ZrP are hydrolyzed in the solvent to have a hydroxy group, which forms a hydrogen bond with an amino group of hexadecylamine. Accordingly, TIP and ZrP become amphiphilic molecules and form micelles. During a process in which hydrolysis and condensation polymerization proceed, the formed micelles are connected with each other at a short distance, and a liquid condensed phase is formed in which a large amount of micelles gather.

Thereafter, the condensation polymerization further occurs, so that the liquid condensed phase is transformed into an inorganic framework. At this time, a portion of the micelle formed becomes a primary particle, a primary particle aggregate formed by aggregation of the micelles becomes a secondary particle (a precursor particle), and a gap between the primary particles becomes a micropore. The precursor particle is required to be calcined to obtain pure TiₓZr₍₁₋ₓ₎O₂ since hexadecylamine (boiling point ≥ 330°C) is present inside. However, calcination at a high temperature causes TiₓZr₍₁₋ₓ₎O₂ to crystallize, and at the same time, the specific surface area decreases. Here, a decrease in specific surface area can be prevented by promoting crystal growth at a low temperature by the solvothermal method and then heating at a high temperature.

TiₓZr₍₁₋ₓ₎O₂ obtained by this synthesis method has a specific surface area larger than that of TiO₂ and ZrO₂. This is due to amorphization of the particles during synthesis through the sol-gel method and the solvothermal method. The inhibition of the crystal growth is caused by mutual inhibition of crystal growth of TiO₂ and ZrO₂ of TiₓZr₍₁₋ₓ₎O₂, which is a solid solution, since ionic radii of Ti⁴⁺ and Zr⁴⁺ are different and crystallite sizes of TiO₂ and ZrO₂ are different.

The specific surface area of the titanium zirconium oxide obtained by the above method is approximately 200 m²/g or more and 500 m²/g or less.

From the viewpoint of enhancing an adsorption function of carbon dioxide in the next step, the specific surface area of the titanium zirconium oxide is preferably 50 m²/g or more.

As described above, TiₓZr₍₁₋ₓ₎O₂ synthesized by the sol-gel method and the solvothermal method has a specific surface area larger than that of TiO₂ or ZrO₂ alone, and can therefore be used as an adsorbent. Since an amount of surface basic sites increases as the specific surface area increases, it is considered that an amount of basic sites in TiₓZr₍₁₋ₓ₎O₂ is greater than that in ZrO₂. That is, TiₓZr₍₁₋ₓ₎O₂ has a large number of basic sites on the surface and has a large specific surface area, and is therefore considered to have high functionality as an adsorbent.

### (Second Step)

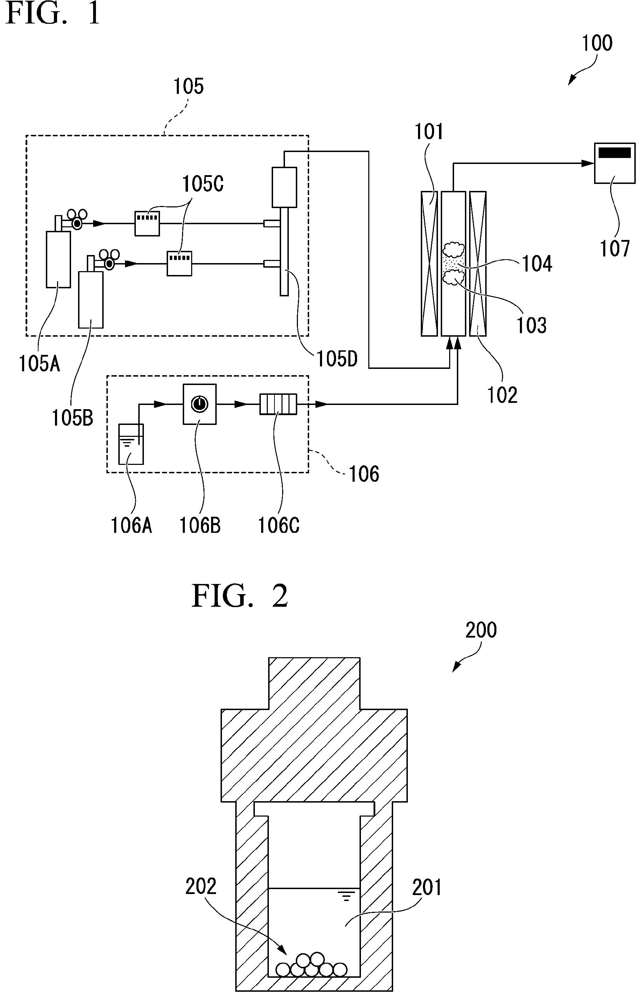

FIG. 1 is a diagram illustrating a configuration example of a gas adsorption device 100. The gas adsorption device 100 mainly includes a tubular electric furnace 101, a reaction tube 102 placed inside the tubular electric furnace 101, glass wool 103 placed inside the reaction tube 102, an oxide 104 that adsorbs carbon dioxide, a first gas supply unit 105 that supplies CO₂ gas and N₂ gas into the reaction tube 102, a second gas supply unit 106 that supplies H₂O gas into the reaction tube 102, and a CO₂ analysis device 107.

Two pieces of glass wool 103 are placed in the reaction tube 102, and the oxide 104 (in this case, the titanium-zirconium oxide) is placed in a space sandwiched between the two pieces of glass wool 103. The first gas supply unit 105 mainly includes a CO₂ source 105A, a N₂ source 105B, a mass flow controller 105C, and a mixer 105D that mixes two gases. The second gas supply unit 106 mainly includes a H₂O source 106A, a pump 106B, and a steam generator 106C.

Carbon dioxide is adsorbed on the surface of the synthesized titanium zirconium oxide using the gas adsorption device 100. Specifically, using the gas adsorption device 100, high pressure is applied to the titanium zirconium oxide and the carbon dioxide in the reaction tube 102 while a temperature is set to a predetermined value. The set temperature is preferably 0°C or higher and 200°C or lower. The applied pressure is preferably 0.1 MPa or more and 1.0 MPa or less.

A general mechanism for adsorbing CO₂ is divided into two mechanisms on a low concentration side and a high concentration side when a width of an adsorbate concentration is wide. On the low concentration side, the adsorbate quickly adsorbs in a monolayer at an adsorption site where an interaction is strong. On the high concentration side, the adsorbate slowly adsorbs in multiple layers in the pore.

In the present embodiment, it is considered that CO₂ is adsorbed to a surface basic site with acid-base interaction as an adsorption driving force on the low concentration side, and CO₂ is adsorbed in multiple layers in the pore on the high concentration side. TiₓZr₍₁₋ₓ₎O₂ has a large specific surface area and a large number of surface basic sites, and can therefore adsorb more CO₂ than TiO₂ and ZrO₂ alone.

### (Third Step)

FIG. 2 is a cross-sectional view of an autoclave 200 used for synthesis of a useful compound. The titanium zirconium oxide having carbon dioxide adsorbed thereto is put into a solvent containing an alkylamine compound such as ethylenediamine, and a heating treatment or the like is performed to synthesize an alkylurea compound (ethylene urea, 2-piperazinone, or the like) which is a useful compound.

Specifically, a solvent 201 containing ethylenediamine is contained in the autoclave 200, and titanium zirconium oxide 202 having carbon dioxide adsorbed thereto is charged thereto. In this state, an inside of the autoclave 200 is heated to react ethylenediamine with carbon dioxide. Here, a heating temperature is preferably 80°C to 200°C, and a heating time is preferably 0.5 h to 24 h.

In the reaction between ethylenediamine and carbon dioxide, at least one of TiO₂, ZrO₂, and CeO₂ may be used as a catalyst for synthesizing ethylene urea.

The heated autoclave is cooled, a compound in the autoclave 200 is filtered to perform solid-liquid separation, and a useful compound such as ethylene urea and 2-piperazinone are obtained in a state of being contained in an extracted liquid phase.

In the autoclave 200 under heating, a two-stage reaction represented by the following equations (3) and (4) occurs. That is, ethylenediaminocarbamic acid is produced by a reaction between ethylenediamine and carbon dioxide that is represented by the equation (3). Subsequently, ethylene urea is synthesized by a dehydration reaction from the ethylenediaminocarbamic acid that is represented by the equation (4). In the reaction of the equation (4), a catalyst that promotes a dehydration reaction is preferably used.

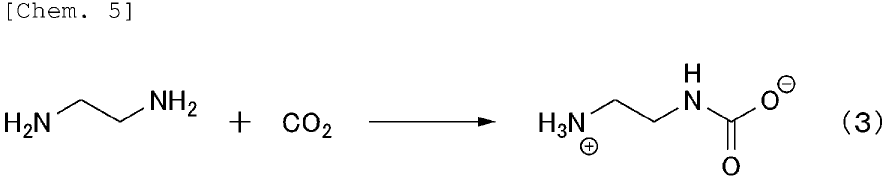

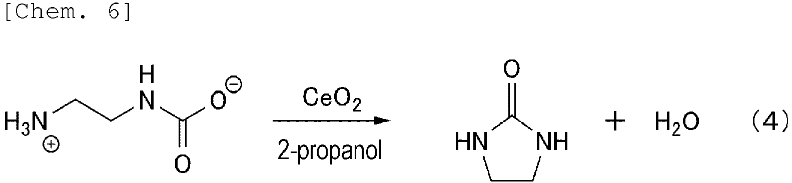

As described above, in the carbon dioxide utilization method of the present embodiment, carbon dioxide in a state of being adsorbed on a solid substance is reacted with ethylenediamine in the solvent. Unlike blowing gaseous carbon dioxide, vaporization of ethylenediamine does not occur, so that carbon dioxide can be reacted with ethylenediamine with high efficiency, and thus effective utilization can be achieved. By using, as a solid substance, titanium zirconium oxide having a large specific surface area and a large number of surface basic sites, a large amount of carbon dioxide can be adsorbed and a large amount of carbon dioxide can be supplied to a reaction field with ethylenediamine, so that a useful compound can be efficiently produced.

### <Second Embodiment>

In a carbon dioxide supply conversion agent and a carbon dioxide utilization method according to a second embodiment of the present invention, a titanium zirconium oxide represented by a composition formula TiₓZr_{y}Ce_{z}O₂, where 0 < x < 1, 0 < y < 1, and 0 < z < 1, is used as an oxide that adsorbs carbon dioxide. In addition, the carbon dioxide supply conversion agent and the carbon dioxide utilization method are similar to those of the first embodiment, and at least have the same effects as those of the carbon dioxide supply conversion agent and the carbon dioxide utilization method of the first embodiment.

In a method for producing an alkylurea compound as the carbon dioxide utilization method of the present embodiment, as a raw material of titanium zirconium oxide (TiₓZr_{y}Ce_{z}O₂), for example, nitric acid cerium (III) hexahydrate (CNH) is used along with titanium (IV) isopropoxide (TIP) and zirconium (IV) propoxide (ZrP). Other configurations are the same as those of the first embodiment.

A specific surface area of the titanium zirconium oxide obtained in the present embodiment is approximately 50 m²/g or more and 500 m²/g or less.

The titanium zirconium oxide of the present embodiment has a lower CO₂ supply amount as compared with the titanium zirconium oxide Tiₓ Zr₍₁₋ₓ₎O₂ of the first embodiment due to a solid solution of CeO₂, but can improve reaction efficiency with ethylenediamine.

### <Third Embodiment>

In a carbon dioxide supply conversion agent and a carbon dioxide utilization method according to a third embodiment of the present invention, a Mg-Al-based layered double hydroxide containing Mg and Al as a constituent metal is used as an oxide that adsorbs carbon dioxide. A ratio (Mg/Al) of the number of moles of the Mg contained to the number of moles of the Al contained can be, for example, 2 or more and 4 or less. In addition, the carbon dioxide supply conversion agent and the carbon dioxide utilization method are similar to those of the first embodiment, and at least have the same effects as those of the carbon dioxide supply conversion agent and the carbon dioxide utilization method of the first embodiment.

The Mg-Al-based layered double hydroxide is a layered compound (LDH) in which a positively charged host layer containing Mg²⁺ and Al³⁺ and a guest layer composed of an anion compensating for the positive charge of the host layer and water are alternately stacked. The Mg-Al-based layered double hydroxide has strong basicity and is therefore capable of adsorbing CO₂ well. The Mg-Al-based layered double hydroxide may be doped with Ce. In the Ce-doped Mg-Al-based layered double hydroxide, Ce is present as a part of a constituent metal of the LDH host layer. Alternatively, Ce is present in a form of hydroxide or oxide.

In the carbon dioxide utilization method of the present embodiment, an alkylurea compound can be produced mainly by the following three steps (first step, second step, and third step).

### (First Step)

The Ce-doped Mg-Al-based layered double hydroxide is synthesized using a coprecipitation method, an impregnation method, or the like. Here, a case where the impregnation method is used will be described as an example. First, a reagent of the Mg-Al-based layered double hydroxide is prepared, and calcined (heated) at 400°C to 700°C for 0.5 hours to 12 hours. Subsequently, the calcined reagent is charged into a Ce(NO₃)₃•6H₂O solution and stirred for 0.5 hours to 12 hours while being heated at 10°C to 90°C. Thereafter, the solution is filtered and washed, the synthesized Ce-doped Mg-Al-based layered double hydroxide is dried, and then pulverized to a size of about 0.1 mm in average diameter.

### (Second Step)

Carbon dioxide is adsorbed on a surface of the synthesized Ce-doped Mg-Al-based layered double hydroxide in the same manner as in the first embodiment.

### (Third Step)

The Ce-doped Mg-Al-based layered double hydroxide having carbon dioxide adsorbed thereto is charged into a solvent containing an alkylamine compound such as ethylenediamine by the same method as in the first embodiment, and a heating treatment or the like is performed to synthesize an alkylurea compound (ethylene urea or the like) which is a useful compound.

Since a reaction between the carbon dioxide adsorbed to the oxide of the present embodiment and ethylenediamine is a high temperature and high pressure reaction, ethylene urea (EU) can be directly synthesized without going through ethylenediaminocarbamic acid (EDA-CA) which is an intermediate during the reaction process.

### <Fourth Embodiment>

In a carbon dioxide supply conversion agent and a carbon dioxide utilization method according to a fourth embodiment of the present invention, a Mg-Al-based layered double hydroxide containing Mg and Al as a constituent metal is used as an oxide that adsorbs carbon dioxide. A ratio (Mg/Al) of the number of moles of the Mg contained to the number of moles of the Al contained can be, for example, 2 or more and 4 or less. Other than elements doped into the Mg-Al-based layered double hydroxide, the carbon dioxide supply conversion agent and the carbon dioxide utilization method are similar to those of the first embodiment, and at least have the same effects as those of the carbon dioxide supply conversion agent and the carbon dioxide utilization method of the first embodiment.

The Mg-Al-based layered double hydroxide is a layered compound in which a positively charged host layer containing Mg²⁺ and Al³⁺ and a guest layer composed of an anion compensating for the positive charge of the host layer and water are alternately stacked. The Mg-Al-based layered double hydroxide has strong basicity and is therefore capable of adsorbing CO₂ well. The Mg-Al-based layered double hydroxide may be doped with Zr. In the Zr-doped Mg-Al-based layered double hydroxide, Zr is present as a part of a constituent metal of the LDH host layer. Alternatively, Zr is present in a form of hydroxide or oxide.

In the carbon dioxide utilization method of the present embodiment, an alkylurea compound can be produced mainly by the following three steps (first step, second step, and third step).

### (First Step)

The Zr-doped Mg-Al-based layered double hydroxide is synthesized using a coprecipitation method, an impregnation method, or the like. Here, a case where the impregnation method is used will be described as an example. First, a reagent of the Mg-Al-based layered double hydroxide is prepared, and calcined (heated) at 400°C to 700°C for 0.5 hours to 12 hours. Subsequently, the calcined reagent is charged into a solution and stirred for 0.5 hours to 12 hours while being heated at 10°C to 90°C. Thereafter, the solution is filtered and washed, the synthesized Zr-doped Mg-Al-based layered double hydroxide is dried, and then pulverized to a size of about 0.1 mm in average diameter.

### (Second Step)

Carbon dioxide is adsorbed on a surface of the synthesized Zr-doped Mg-Al-based layered double hydroxide in the same manner as in the first embodiment.

### (Third Step)

The Zr-doped Mg-Al-based layered double hydroxide having carbon dioxide adsorbed thereto is charged into a solvent containing an alkylamine compound such as ethylenediamine by the same method as in the first embodiment, and a heating treatment or the like is performed to synthesize an alkylurea compound (ethylene urea, 2-piperazinone, or the like) which is a useful compound.

### <Fifth Embodiment>

Ce(OH)₄ or Zr(OH)₄ may be used as an oxide that adsorbs carbon dioxide. In a carbon dioxide supply conversion agent and a carbon dioxide utilization method according to a fifth embodiment of the present invention, an example will be given in which Ce(OH)₄ is used as the oxide that adsorbs carbon dioxide. In addition, the carbon dioxide supply conversion agent and the carbon dioxide utilization method are similar to those of the first embodiment, and at least have the same effects as those of the carbon dioxide supply conversion agent and the carbon dioxide utilization method of the first embodiment.

In the carbon dioxide utilization method of the present embodiment, an alkylurea compound can be produced mainly by the following three steps (first step, second step, and third step).

### (First Step)

Ce(OH)₄ is synthesized using a coprecipitation method. First, a Ce(NO₃)₃ solution is put in a five-necked flask, and then a NaOH solution is further added dropwise thereto to achieve a predetermined pH (9 to 12), followed by stirring. The obtained suspension is suction-filtered, and washed with ion-exchanged water until the filtrate becomes neutral. The obtained product is dried under reduced pressure and pulverized to obtain a synthesized Ce(OH)₄.

### (Second Step)

The synthesized Ce(OH)₄ is packed on glass wool in a reaction tube, and the reaction tube is set in an electric furnace set at 10°C to 100°C. A carbon dioxide-nitrogen mixed gas is circulated in the electric furnace. A CO₂ concentration at an outlet of the electric furnace is monitored with a gas analyzer, and CO₂ is adsorbed onto Ce(OH)₄ until a breakthrough occurs.

### (Third Step)

The Ce(OH)₄ having CO₂ adsorbed thereto is charged into an autoclave together with 2-propanol and EDA, and the mixture is heated at 80°C to 160°C for 2 hours to 48 hours for reaction. The heated autoclave is cooled at room temperature, the reaction product is extracted from the autoclave, and solid-liquid separation is performed by filtration to obtain synthesized ethylene urea.

### Example

Effects of the invention will be more clearly understood based on the following examples. The invention is not limited to the following examples, and can be appropriately modified without departing from the scope of the invention.

### (Example 1)

Titanium zirconium oxides Ti_{0.7}Zr_{0.3}O₂, Ti_{0.5}Zr_{0.5}O₂, and Ti_{0.3}Zr_{0.7}O₂, ZrO₂, TiO₂, and CeO₂ were synthesized by the first step in the above first embodiment. Specifically, synthesis was performed in the following procedure.

### [Synthesis of TiₓZr₍₁₋ₓ₎O₂, and ZrO₂]

### (Sol-Gel Method)

To 790 mL of ethanol, 7.95 g of hexadecylamine (HDA), 3.2 mL of a 0.1 M potassium chloride (KCl) solution, and 5.44 mL of ultrapure water were added, followed by stirring with a magnetic stirrer until hexadecylamine was completely dissolved.

After the dissolution, a mixed solution of 10 mL of ethanol, titanium (IV) isopropoxide (TIP) (Ti[OCH(CH₃)₂]₄), and zirconium (IV) propoxide (ZrP) (Zr(OCH₂CH₂CH₃)₄) was added while stirring, and the stirring was continued for another 2 minutes. A mixing ratio of TIP and ZrP in the mixed solution is shown in Table 1.

The stirred solution was allowed to stand in a dark place for 24 h and subjected to solid-liquid separation using a centrifugal separation device to obtain a TiₓZr₍₁₋ₓ₎ O₂ precursor.

**[Table 1]**

| Reagent | Ti_{0.7}Zr_{0.3}O₂ | Ti_{0.5}Zr_{0.5}O₂ | Ti_{0.3}Zr_{0.7}O₂ | ZrO₂ |
|---|---|---|---|---|
| TIP [mL] | 12.71 | 9.08 | 5.45 | 0 |
| ZrP [mL] | 8.00 | 13.34 | 18.67 | 26.67 |

### (Solvothermal Method)

Into a 45 mL autoclave, 1.6 g of TiₓZr₍₁₋ₓ₎O₂ precursor was charged, and 20 mL of ethanol and 10 mL of ion-exchanged water were added thereto, followed by stirring for about 30 minutes. After the stirring, the mixture was heated at 160°C for 24 h, and cooled at room temperature, and then solid-liquid separation is performed by centrifugal separation (10000 rpm for 15 min) while washing the synthesized solid with ethanol. The ethanol washing was repeated three times. The washed solid was dried under reduced pressure at 40°C, and the dried solid was heated to 500°C in a muffle furnace over 5 h and calcined at 500°C for 2 h to obtain TiₓZr₍₁₋ₓ₎O₂.

### [Synthesis of TiO₂]

### (Sol-Gel Method)

To 790 mL of ethanol, 7.95 g of hexadecylamine (HDA) and 3.2 mL of a 0.1 M potassium chloride (KCl) solution (concentration adjusted in a measuring flask) were added, followed by stirring with a magnetic stirrer until hexadecylamine was completely dissolved.

After the dissolution, a mixed solution of 10 mL of ethanol and 18.10 mL of TIP was added while stirring, and the stirring was continued for another 2 minutes. The stirred solution was allowed to stand in a dark place for 24 h and subjected to solid-liquid separation by centrifugal separation (10000 rpm for 15 min) while washing with ethanol to obtain a TiO₂ precursor.

### (Solvothermal Method)

Into a 45 mL autoclave, 1.6 g of the precursor particles were charged, and 20 mL of ethanol, 10 mL of ion-exchanged water, and further 0.572 mL of aqueous ammonia were added thereto, followed by stirring for about 30 minutes. After heating at 160°C for 24 h and cooling at room temperature, solid-liquid separation was performed by centrifugal separation (10000 rpm for 15 min) while washing the synthesized solid with ethanol. The ethanol washing was repeated three times. The washed solid was dried under reduced pressure at 40°C, and the dried solid was heated to 500°C in a muffle furnace over 5 h and calcined at 500°C for 2 h to obtain TiO₂.

### [Synthesis of CeO₂]

### (Sol-Gel Method)

To 790 mL of ethanol, 7.95 g of hexadecylamine (HDA), 3.2 mL of a 0.1 M potassium chloride (KCl) solution (concentration adjusted in a measuring flask), and 5.44 mL of ultrapure water were added, followed by stirring with a magnetic stirrer until hexadecylamine was completely dissolved.

After the dissolution, a mixed solution of 10 mL of ethanol and cerium (III) nitrate hexahydrate was added while stirring, and the stirring was continued for another 2 minutes. The stirred solution was allowed to stand in a dark place for 24 h and subjected to solid-liquid separation by centrifugal separation (10000 rpm for 10 min) while washing with ethanol to obtain a CeO₂ precursor. The ethanol washing was repeated three times.

### (Solvothermal Method)

Into a 45 mL autoclave, 1.6 g of precursor particles were charged, and 10 mL of ethanol and 10 mL of ion-exchanged water were added thereto, followed by stirring for about 30 minutes. After heating at 160°C for 24 h and cooling at room temperature, solid-liquid separation was performed by centrifugal separation (10000 rpm for 10 min) while washing the synthesized solid with ethanol. The ethanol washing was repeated three times. The washed solid was dried under reduced pressure at 40°C, and the dried solid was heated to 500°C in a muffle furnace over 5 h and calcined at 500°C for 2 h to obtain CeO₂.

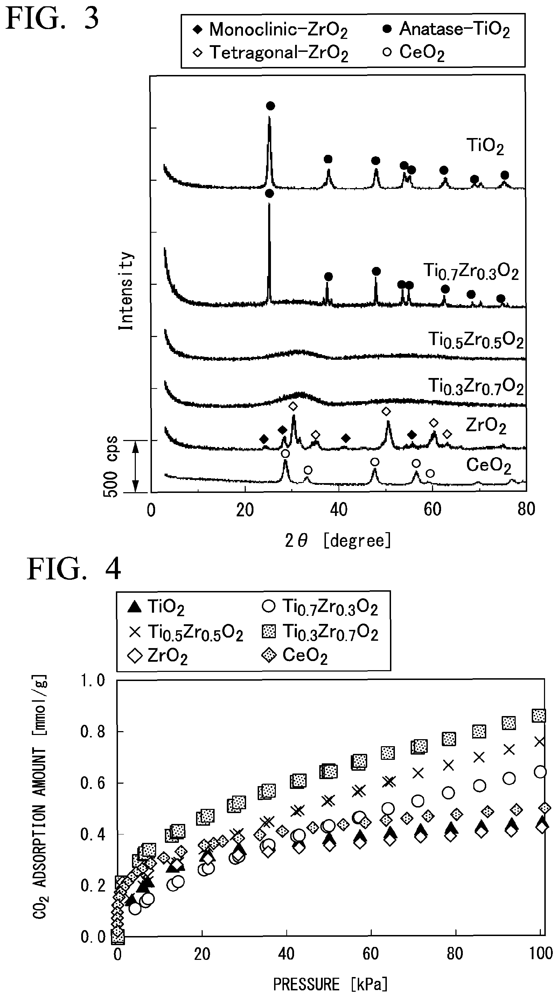

The synthesized titanium zirconium oxide Ti_{0.7}Zr_{0.3}O₂, Ti_{0.5}Zr_{0.5}O₂, and Ti_{0.3}Zr_{0.7}O₂, ZrO₂, TiO₂, and CeO₂ were subjected to X-ray diffraction measurement (XRD, Cu Kα ray) . FIG. 3 is a graph illustrating an XRD pattern obtained by the measurement.

TiO₂ and ZrO₂ synthesized by a combination of the sol-gel method and the solvothermal method have an anatase TiO₂ phase, and monoclinic and tetragonal ZrO₂ phases, respectively. A peak corresponding to CeO₂ alone is also observed in CeO₂ synthesized by the same method. In Ti_{0.7}Zr_{0.3}O₂, a peak corresponding to the anatase TiO₂ phase is observed, but peaks corresponding to the monoclinic and tetragonal ZrO₂ phases at 2θ = 25° to 40° are broadened. On the other hand, in Ti_{0.5}Zr_{0.5}O₂ and Ti_{0.3}Zr_{0.7}O₂, clear diffraction lines are not observed, suggesting that they are amorphous. This is considered to be because Ti⁴⁺ and Zr⁴⁺ mutually prevent crystal growth of TiO₂ and ZrO₂ in a crystal growth process in a system where Zr⁴⁺ is present in an amount equal to or greater than that of Ti⁴⁺.

A specific surface area and a pore volume of the synthesized titanium zirconium oxides Ti_{0.7}Zr_{0.3} O₂, Ti_{0.5}Zr_{0.5}O₂, and Ti_{0.3}Zr_{0.7}O₂, ZrO₂, TiO₂, and CeO₂ were calculated based on N₂ adsorption data. The calculation result is shown in Table 2.

**[Table 2]**

| Sample | Specific surface area [m²/g] | Pore volume [cm³/g] |
|---|---|---|
| TiO₂ | 89 | 0.31 |
| Ti_{0.7}Zr_{0.3}O₂ | 125 | 0.22 |
| Ti_{0.5}Zr_{0.5}O₂ | 352 | 0.43 |
| Ti_{0.3}Zr_{0.7}O₂ | 192 | 0.24 |
| ZrO₂ | 70 | 0.17 |
| CeO₂ | 85 | 0.093 |

TiₓZr₍₁₋ₓ₎O₂ has a specific surface area larger than that of TiO₂ and ZrO₂ alone, and among them, Ti_{0.5}Zr_{0.5}O₂ has the largest value of 352 m²/g. As described above, it is considered that TiO₂ and ZrO₂ mutually prevent the crystal growth, resulting in a smaller particle size and a larger specific surface area.

Carbon dioxide was adsorbed to surfaces of the synthesized titanium zirconium oxides Ti_{0.7}Zr_{0.3}O₂, Ti_{0.5}Zr_{0.5}O₂, and Ti_{0.3}Zr_{0.7}O₂, ZrO₂, TiO₂, and CeO₂, respectively, and an adsorption amount (an adsorption isotherm) was measured using a gas adsorption device (BELSORP MINIX manufactured by MicrotracMRB). Specifically, the measurement was performed in the following procedure.

Into separate sample tubes, 0.02 g to 0.15 g of TiO₂, ZrO₂, TiₓZr₍₁₋ₓ₎O₂, and CeO₂ were charged, respectively. Subsequently, a sample portion of each sample tube was heated at 200°C for 2 h while evacuating the sample portion to remove moisture adhering to the surface. Subsequently, each sample tube was set in a gas adsorption device, and a CO₂ adsorption isotherm was measured. A weight of the sample tube after heating was measured, and a weight of the sample tube in an empty state was subtracted to determine a sample weight.

FIG. 4 is a graph (an adsorption isotherm) illustrating a result of the measurement. A horizontal axis in the graph represents pressure [kPa], and a vertical axis in the graph represents a CO₂ adsorption amount [mmol/g]. There is no significant difference in adsorption amount between TiO₂ and ZrO₂ alone, and the adsorption amount is about 0.40 mmol/g at CO₂ pressure of 100 kPa. A CO₂ adsorption amount of CeO₂ is slightly higher than a CO₂ adsorption amount of TiO₂ and ZrO₂, and is about 0.50 mmol/g at CO₂ pressure of 100 kPa. On the other hand, in TiₓZr₍₁₋ₓ₎O₂, a CO₂ adsorption amount increases greatly in proportion to the pressure. In particular, Ti_{0.3}Zr_{0.7} O₂ exhibits the highest adsorption amount, increases to 0.95 mmol/g at pressure of 100 kPa, which is more than twice the adsorption amount of TiO₂ and ZrO₂.

As shown in the following equation (5), Ti_{0.7}Zr_{0.3}O₂, Ti_{0.5}Zr_{0.5}O₂, Ti_{0.3}Zr_{0.7}O₂, ZrO₂, TiO₂, and CeO₂ after CO₂ adsorption were reacted with EDA.

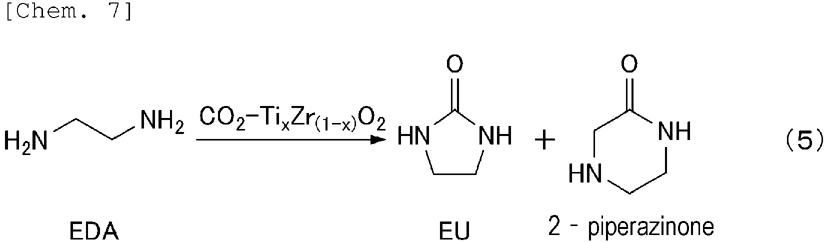

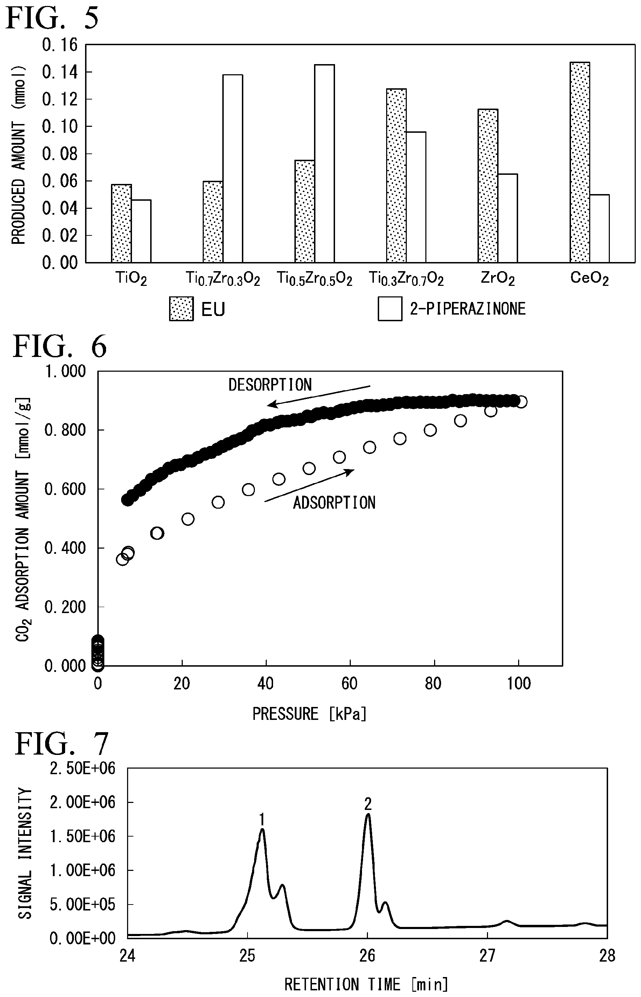

FIG. 5 is a graph illustrating a result of this reaction. In either case, EU and 2-piperazinone can be produced by the reaction between EDA and CO₂ adsorbed on a sample. When a total produced amount of EU and 2-piperazinone is taken into account, it can be seen that TiₓZr₍₁₋ₓ₎O₂ produces more than ZrO₂ and TiO₂. It is considered that TiₓZr₍₁₋ₓ₎O₂ has a total CO₂ adsorption amount larger than that of ZrO₂ and TiO₂, and a larger amount of CO₂ is used for the reaction with EDA. From these results, it can be seen that with respect to effective utilization of CO₂, TiₓZr₍₁₋ₓ₎O₂ is suitable as a CO₂ supply source.

With respect to TiₓZr₍₁₋ₓ₎O₂, when each product amount is taken into account, it can be seen that the larger the proportion of Zr in the reaction product, the larger the produced amount of EU, which is a main product. On the other hand, it can be seen that the smaller the proportion of Zr in the reaction product, the smaller the produced amount of 2-piperazinone, which is a by-product. From these results, it can be seen that the higher the proportion of Zr in TiₓZr₍₁₋ₓ₎O₂, the higher the selectivity for EU production. It can be seen that the total produced amount is the largest and the EU selectivity is also the highest in the case of CeO₂.

### (Example 2)

Titanium zirconium oxide Ti_{0.3}Zr_{0.7}O₂ was synthesized by the same procedure as in Example 1, and an adsorption amount and a desorption amount of carbon dioxide were measured. Pressure to be applied was increased from atmospheric pressure to 100 kPa, and then returned to atmospheric pressure.

FIG. 6 is a graph (an adsorption-desorption isotherm) illustrating a result of the measurement. A horizontal axis and a vertical axis in the graph are the same as those in FIG. 4. Since a desorption isotherm is above a CO₂ adsorption isotherm without overlapping it, it can be said that there is a tendency for CO₂ to be difficult to desorb. A current amount of CO₂ present at atmospheric pressure is about 400 ppm, which is converted to a pressure of 22.5 kPa using a gas state equation. An amount of CO₂ adsorbed on a surface of Ti_{0.3}Zr_{0.7}O₂ at 22.5 kPa is approximately 0.7 mmol/g. The reason why the adsorption isotherm and the desorption isotherm did not overlap with each other is considered to be that acid-base interaction acts between CO₂ and a surface basic site, making desorption difficult, and that a large amount of CO₂ was adsorbed into pores, which were small in diameter, making desorption difficult.

### (Example 3)

Titanium zirconium oxide Ti_{0.3}Zr_{0.7}O₂ synthesized by the same procedure as in Example 1 and having CO₂ adsorbed thereto was reacted with ethylenediamine according to the third step in the above embodiment to synthesize a compound. Specifically, synthesis was performed in the following procedure.

Into an autoclave, 0.4 g of Ti_{0.3}Zr_{0.7}O₂ after CO₂ adsorption, 5 mmol of ethylenediamine, and 25 mL of 2-propanol were charged, followed by heating at 160°C for 24 h. The heated autoclave was cooled at room temperature, and a compound in the autoclave 200 was filtered to perform solid-liquid separation.

To an extracted liquid phase (a post-reaction solution), 0.037 g of tert-butyl alcohol was added as a standard substance, and analysis was performed by a chromatography mass analysis device (GC-MS). FIG. 7 is a graph (a total ion chromatogram) illustrating a result of the analysis. A horizontal axis in the graph represents a retention time [min], and a vertical axis in the graph represents a signal intensity.

Peaks 1 and 2 of the signal intensity were detected at positions where the retention time was 25.1 minutes and 26.0 minutes. Therefore, chromatography mass spectrometry was performed on each of ethylene urea alone and 2-piperazinone alone, and a peak of the signal intensity for ethylene urea alone was almost identical to the peak 1, and a peak of the signal intensity for 2-piperazinone alone was almost identical to the peak 2. When mass spectrum patterns of the peaks 1 and 2 were analyzed, the mass spectrum patterns of the peaks 1 and 2 were almost identical to mass spectrum patterns of ethylene urea and 2-piperazinone, respectively. From these results, it can be seen that ethylene urea and 2-piperazinone, which are a useful compound, are synthesized by a reaction of CO₂ with ethylenediamine.

### (Example 4)

Titanium zirconium oxide Ti_{0.27}Zr_{0.63}Ce_{0.10}O₂, Ti_{0.24}Zr_{0.56}Ce_{0.20}O₂, Ti_{0.21}Zr_{0.49}Ce_{0.30}O₂, and Ti_{0.18}Zr_{0.42}Ce_{0.40}O₂ were synthesized by the first step in the above embodiment. Specifically, synthesis was performed in the following procedure.

### (Sol-Gel Method)

To 790 mL of ethanol, 7.95 g of hexadecylamine (HDA) and 3.2 mL of a 0.1 M potassium chloride (KCl) solution were added, followed by stirring with a magnetic stirrer until hexadecylamine was completely dissolved.

After the dissolution, a mixed solution of 10 mL of ethanol, cerium (III) nitrate hexahydrate (CNH), titanium (IV) isopropoxide (TIP), and zirconium (IV) propoxide (ZrP) was added while stirring, and the stirring was continued for another 2 minutes. A mixing ratio of CNH, TIP, and ZrP at this time is shown in Table 3. A proportion of Ce to the whole was adjusted to change in a range of z = 0.1 to 0.4 while maintaining Ti: Zr = 3:7. The stirred solution was allowed to stand in a dark place for 24 h and subjected to solid-liquid separation using centrifugal separation to obtain a TiₓZr_{y}Ce_{z}O₂ precursor.

**[Table 3]**

| Reagent | Ti_{0.27}Zr_{0.63}Ce_{0.10}O₂ | Ti_{0.24}Zr_{0.56}Ce_{0.20}O₂ | Ti_{0.21}Zr_{0.49}Ce_{0.30}O₂ | Ti_{0.18}Zr_{0.42}Ce_{0.40}O₂ |
|---|---|---|---|---|
| TIP [mL] | 4.90 | 4.36 | 3.81 | 2.72 |
| ZrP [mL] | 16.80 | 14.94 | 13.07 | 9.33 |
| CNH [mL] | 2.58 | 5.17 | 11.20 | 12.92 |

### (Solvothermal Method)

Into a 45 mL autoclave, 1.6 g of the precursor particles were charged, and 20 mL of ethanol and 10 mL of ion-exchanged water were added thereto, followed by stirring for about 30 minutes. After the stirring, the mixture was heated at 160°C for 24 h, and cooled at room temperature, and then solid-liquid separation is performed by centrifugal separation (10000 rpm for 15 min) while washing the synthesized solid with ethanol. The ethanol washing was repeated three times.

The washed solid was dried under reduced pressure at 40°C, and the dried solid was calcined in a muffle furnace at 500°C for 5 h to obtain TiₓZr_{y}Ce_{z}O₂.

Carbon dioxide was adsorbed on surfaces of the synthesized titanium zirconium oxide Ti_{0.27}Zr_{0.63}Ce_{0.10}O₂, Ti_{0.24}Zr_{0.56}Ce_{0.20}O₂, Ti_{0.21}Zr_{0.49}Ce_{0.30}O₂, and Ti_{0.18}Zr_{0.42}Ce_{0.40}O₂, respectively, by the same procedure as in Example 1, and an adsorption amount (an adsorption isotherm) was measured using a gas adsorption device (BELSORP MINIX manufactured by MicrotracMRB).

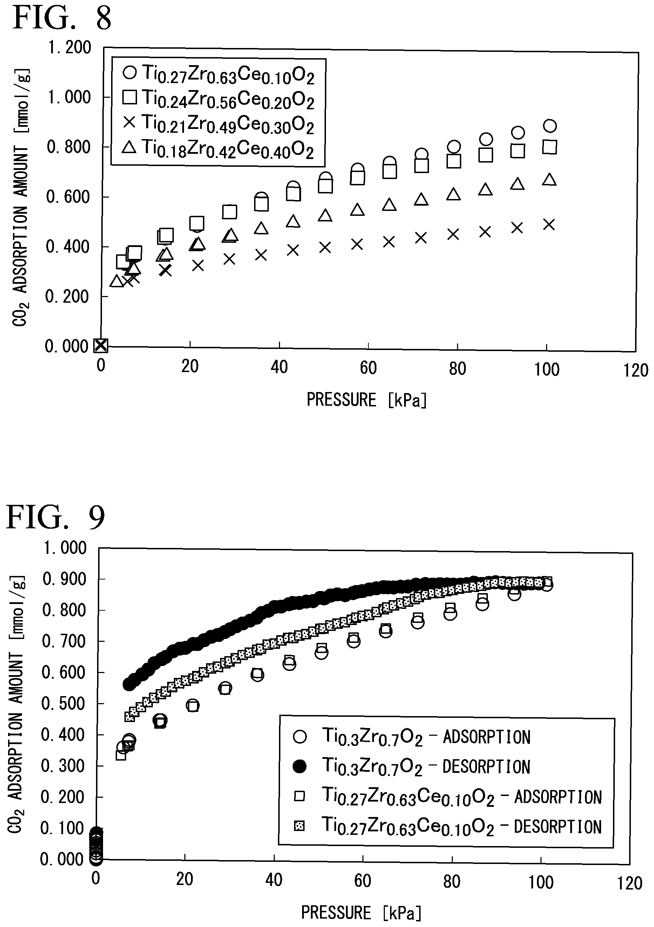

FIG. 8 is a graph (an adsorption isotherm) illustrating a result of the measurement. A horizontal axis in the graph represents pressure [kPa], and a vertical axis in the graph represents a CO₂ adsorption amount [mmol/g]. A CO₂ adsorption amount was the largest in Ti_{0.27}Zr_{0.63}Ce_{0.10}O₂, and a difference with Ti_{0.3}Zr_{0.7}O₂ in Example 1 (FIG. 4) was small. Since a specific surface area and a pore volume tend to decrease when a solid solution proportion of CeO₂ increases, it is considered that the CO₂ adsorption amount is smaller as compared with Ti_{0.3}Zr_{0.7}O₂ which does not use CeO₂.

In a sample with a CeO₂ solid solution proportion of 30%, such a tendency for the CO₂ adsorption amount to decrease was observed, and conversely, in a sample with a CeO₂ solid solution proportion of 40%, a tendency for the CO₂ adsorption amount to be greater as compared with Ti_{0.3}Zr_{0.7}O₂ was observed. It is considered to be because there is no significant difference in specific surface area, but a proportion of ZrO₂ and CeO₂ having a basic site to TiO₂ having no basic site increases, and the CO₂ adsorption amount due to the acid-base interaction increases.

### (Example 5)

Titanium zirconium oxide Ti_{0.3}Zr_{0.7}O₂, Ti_{0.27}Zr_{0.63}Ce_{0.10}O₂ was synthesized by the same procedure as in Example 4, and an adsorption amount and a desorption amount of carbon dioxide were measured. Pressure to be applied was increased from atmospheric pressure to 100 kPa, and then returned to atmospheric pressure.

FIG. 9 is a graph illustrating a result of the measurement. A horizontal axis and a vertical axis in the graph are the same as those in FIG. 6. It can be seen that no significant difference was observed in CO₂ adsorption amount between Ti_{0.3}Zr_{0.7}O₂ and Ti_{0.27}Zr_{0.63}Ce_{0.10}O₂, but for the desorption amount, CO₂ tends to be more easily desorbed from Ti_{0.27}Zr_{0.63}Ce_{0.10}O₂. This difference is considered to be due to a size of a pore. Most of the adsorbed CO₂ is adsorbed to the pore, and the larger the pore diameter, the more easily the molecules present in the pore are released outside the pore. Ti_{0.3}Zr_{0.7}O₂ has a pore diameter of about 2 nm to 3 nm, whereas Ti_{0.27} Zr_{0.63} Ce_{0.10} O₂ has a large number of pores of 5 nm or more. Since the molecular diameter of CO₂ is very small at 0.224 nm, it is considered that this slight difference affects the desorption amount. A CO₂ concentration under air is 400 ppm, and a residual adsorption amount of CO₂ at 400 ppm is about 0.55 mmol/g.

### (Example 6)

Titanium zirconium oxide Ti_{0.2}-Zr_{0.63}Ce_{0.10}O₂ synthesized by the same procedure as in Example 4 and having CO₂ adsorbed thereto was reacted with ethylenediamine according to the third step in the above embodiment to synthesize a compound.

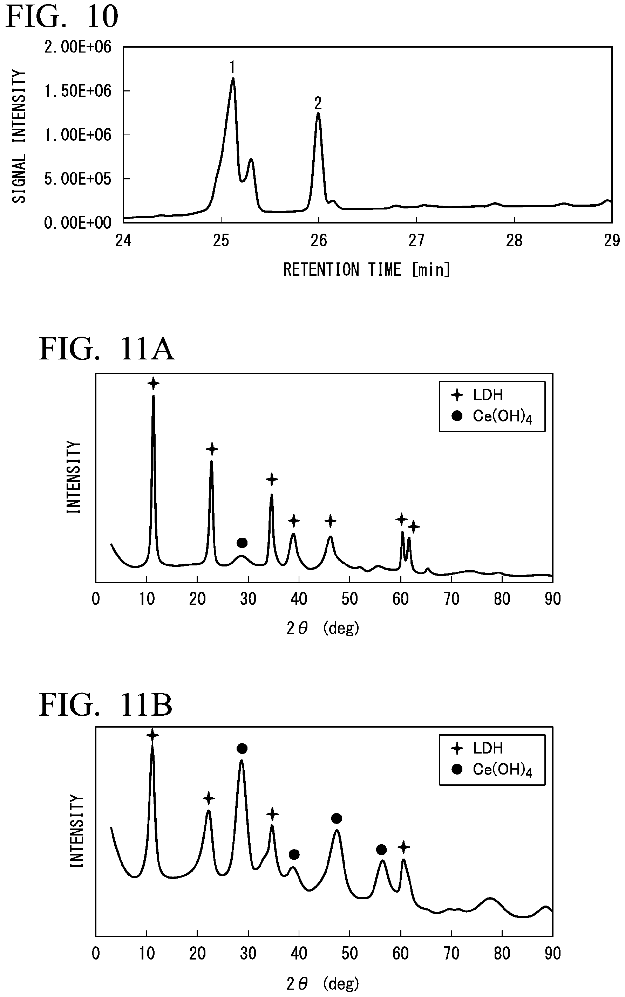

To a liquid phase (a post-reaction solution) extracted by solid-liquid separation, 0.037 g of tert-butyl alcohol was added as a standard substance, and analysis was performed by a chromatography mass analysis device (GC-MS). FIG. 10 is a graph (a total ion chromatogram) illustrating a result of the analysis. A horizontal axis and a vertical axis in the graph are the same as those in FIG. 7.

As in the case of synthesis of ethylene urea in Example 3, the peaks 1 and 2 were observed at positions of retention times of 25.1 minutes and 26.0 minutes where peaks of ethylene urea and 2-piperazinone were observed, respectively. When mass spectra of the peaks 1 and 2 were analyzed, mass spectrum patterns of the peaks 1 and 2 were almost identical to those of ethylene urea and 2-piperazinone. From this, it can be seen that Ti_{0.27}Zr_{0.63}Ce_{0.1}O₂ also functions as a supply source of CO₂ similar to TiₓZr₍₁₋ₓ₎O₂. From these results, it can be seen that ethylene urea and 2-piperazinone, which are a useful compound, are synthesized.

### (Example 7)

A Ce-doped Mg-Al-based layered double hydroxide was synthesized by the first step in the above third embodiment. Specifically, synthesis was performed in the following procedure.

First, a reagent of a Mg-Al-based layered double hydroxide was prepared and calcined at 500°C for 18 hours. Subsequently, the calcined reagent was put into a Ce(NO₃)₃•6H₂O solution and stirred in a state of being heated at 60°C for 6 hours. Amounts of the reagent and the solution were adjusted such that a molar ratio (Ce/Al) of Ce to Al was 0.1. Thereafter, the solution was filtered and washed, the synthesized Ce-doped Mg-Al-based layered double hydroxide was dried, and then pulverized to a size of about 0.1 mm in average diameter.

### (Example 8)

In the same manner as in Example 7, a Ce-doped Mg-Al-based layered double hydroxide was synthesized and pulverized. Amounts of a reagent and a solution were adjusted such that a molar ratio (Ce/Al) of Ce to Al was 0.5.

The Ce-doped Mg-Al-based layered double hydroxide synthesized in Examples 7 and 8 was subjected to X-ray diffraction measurement. FIGS. 11A and 11B are graphs illustrating measurement results in Examples 7 and 8, respectively. A horizontal axis in the graph represents a diffraction angle, and a vertical axis in the graph represents a diffraction intensity.

It can be seen that in both Examples 7 and 8, the presence of peaks indicated by cross symbols indicates that an LDH structure is maintained even in a Ce-doped state. It can be seen that in both Examples 7 and 8, the presence of peaks indicated by black circles indicates the presence of Ce(OH)₄ in the LDH structure. From a comparison between Examples 7 and 8, it can be seen that as the molar ratio (Ce/Al) increases, a peak derived from Ce(OH)₄ appears more remarkably.

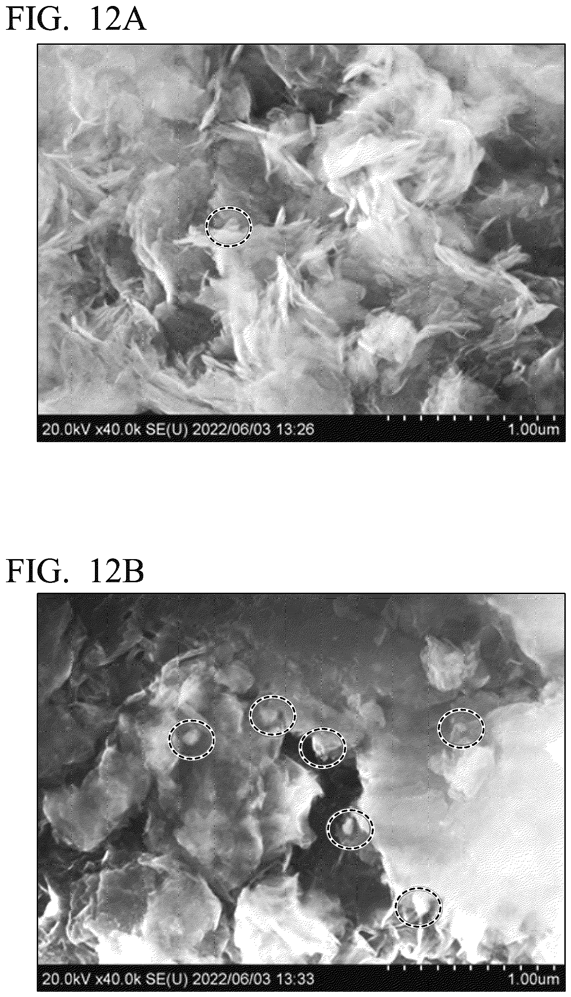

An SEM image of the Ce-doped Mg-Al-based layered double hydroxide synthesized in Examples 7 and 8 was obtained. FIGS. 12A and 12B are SEM images in Examples 7 and 8, respectively. A position of Ce(OH)₄ is indicated by a circle.

In both Examples 7 and 8, the LDH structure and the presence of Ce(OH)₄ in particulate form can be confirmed. Since the number of Ce(OH)₄ in Example 8 is larger than the number of Ce(OH)₄ in Example 7, it can be seen that formation of Ce(OH)₄ proceeds as the molar ratio (Ce/Al) increases.

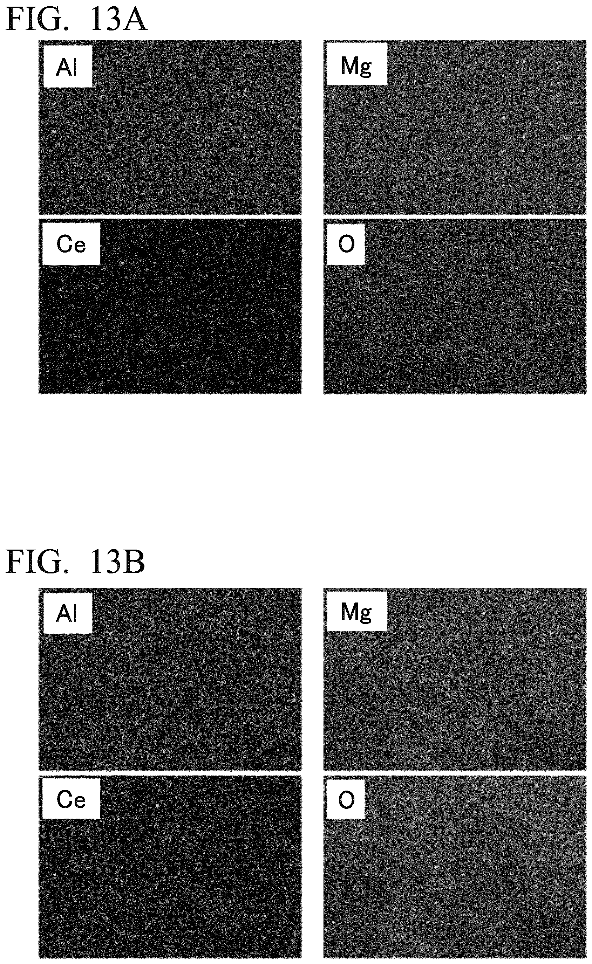

Elemental mapping of the Ce-doped Mg-Al-based layered double hydroxide synthesized in Examples 7 and 8 was performed. FIGS. 13A and 13B illustrate distributions of Al, Mg, Ce and O in Examples 7 and 8, respectively.

It can be seen that Ce is distributed evenly in both Examples 7 and 8, but a distribution density of Ce is higher in Example 8 as compared with Example 7.

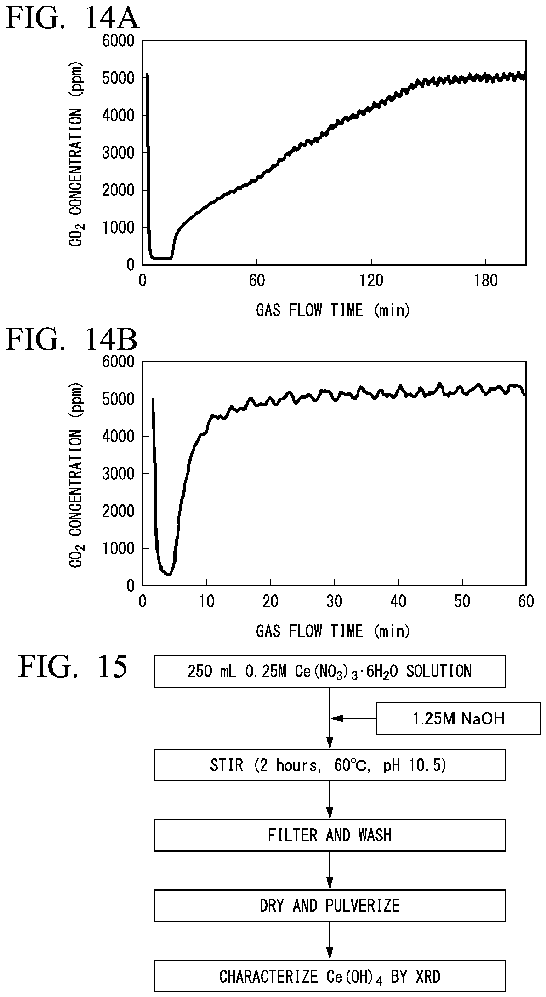

The Ce-doped Mg-Al-based layered double hydroxide synthesized in Examples 7 and 8 was subjected to carbon dioxide gas adsorption measurement. FIGS. 14A and 14B are graphs illustrating measurement results in Examples 7 and 8, respectively. A horizontal axis in the graph represents a gas flow time, and a vertical axis in the graph represents a CO₂ concentration.

In Example 7, a breakthrough state was reached 180 minutes after start of carbon dioxide flow, whereas in Example 8, a breakthrough state was reached 60 minutes after start of carbon dioxide flow. An adsorption capacity of the Ce-doped Mg-Al-based layered double hydroxide in Example 7 was 0.455 mmol/g, whereas an adsorption capacity of the Ce-doped Mg-Al-based layered double hydroxide in Example 8 was 0.0514 mmol/g.

### (Example 9)

A Ce-doped Mg-Al-based layered double hydroxide was synthesized in the same manner as in Example 7, and then ethylene urea and 2-piperazinone were synthesized by the second step and the third step in the above third embodiment.

In the second step, carbon dioxide was adsorbed on a surface of the synthesized Ce-doped Mg-Al-based layered double hydroxide by using a gas adsorption device. A set temperature in a reaction tube was set to 30°C, and pressure applied to the reaction tube was set to 0.1 MPa.

In the third step, a solvent containing 0.3 g (5 mmol) of ethylenediamine and 25 ml of 2-propanol was contained in an autoclave, and 0.4 g of Ce-doped Mg-Al-based layered double hydroxide having carbon dioxide adsorbed thereto was added thereto. In this state, an inside of the autoclave was heated to react ethylenediamine with carbon dioxide. Here, a heating temperature was 160°C and a heating time was 24 h.

### (Example 10)

Ethylene urea and 2-piperazinone were synthesized in the same manner as in Example 9. In the first step, amounts of a reagent and a solution were adjusted such that a molar ratio (Ce/Al) of Ce to Al was 0.5.

Produced amounts of ethylene urea and 2-piperazinone in Examples 9 and 10 are shown in Table 4. From these results, it can be seen that carbon dioxide adsorbed to the Ce-doped Mg-Al-based layered double hydroxide can be used for a synthesis reaction of ethylene urea and 2-piperazinone, which are a useful compound.

**[Table 4]**

| | Molar ratio (Ce/Al) | Produced amount of ethylene urea | Produced amount of 2-piperazinone |
|---|---|---|---|
| Example 9 | 0.1 | 0.048 | 0.025 |
| Example 10 | 0.5 | 0.032 | 0.019 |

### (Example 11)

Ce(OH)₄ was synthesized by the first step in the above fifth embodiment. Specifically, the synthesis was performed according to a procedure shown in FIG. 15.

Into a five-necked flask, 250 mL of a 0.25 mol/L Ce(NO₃)₃ solution was put, and the pH was maintained at 10.5 by dropwise addition of a 1.25 mol/L NaOH solution, followed by stirring at 60°C for 2 hours.

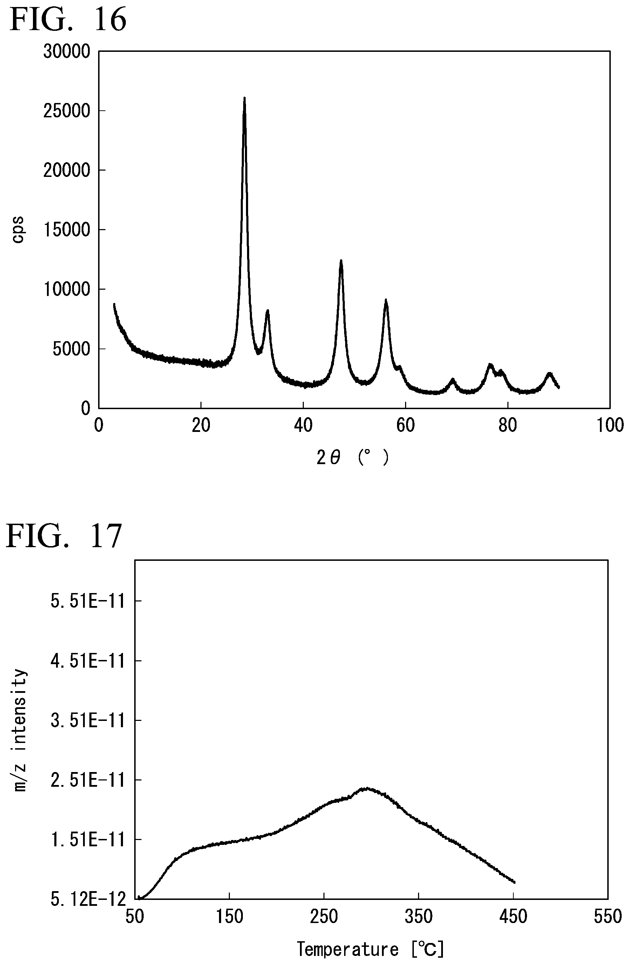

The suspension obtained after stirring was suction-filtered, and washed with ion-exchanged water until the filtrate becomes neutral. The obtained product was dried under reduced pressure at 40°C for 40 hours and pulverized to obtain a synthesized Ce(OH)₄. A phase of the synthesized Ce(OH)₄ was identified using an X-ray diffractometer. FIG. 16 is a graph illustrating the obtained X-ray diffraction pattern. It can be confirmed that the X-ray diffraction pattern has a peak derived from Ce(OH)₄.

### (Example 12)

Ce(OH)₄ was synthesized in the same manner as in Example 11, and then CO₂ was adsorbed on the Ce(OH)₄ by the second step in the above fifth embodiment.

In the second step, 1.0 g of synthesized Ce(OH)₄ was packed on glass wool in a reaction tube (inner diameter 16 mm), and the reaction tube was set in a tubular electric furnace set at 30°C. A carbon dioxide-nitrogen mixed gas was circulated in the tubular electric furnace at a flow rate of 201 mL/min (linear velocity 1.0 m/min). A CO₂ concentration at an outlet of the tubular electric furnace is monitored with a gas analyzer, and CO₂ is adsorbed onto Ce(OH)₄ until a breakthrough occurs.

A CO₂ adsorption amount of Ce(OH)₄ was measured using a temperature programmed desorption (TPD) device. FIG. 17 is a graph illustrating a CO₂-TPD profile. A horizontal axis in the graph represents a temperature, and a vertical axis in the graph represents a CO₂desorption amount (intensity). As a result of integral calculation from the CO₂-TPD profile, the CO₂ adsorption amount of Ce(OH)₄ was 0.512 mmol/g.

### (Examples 13 to 17)

As in Example 12, CO₂ was adsorbed onto Ce(OH)₄, and then ethylene urea was synthesized by the third step in the above fifth embodiment.

In the third step, 25 mL of 2-propanol, 0.4 g of Ce(OH)₄ after CO₂ adsorption, and 0.3 g of EDA were put into an autoclave and heated for 24 hours. Reaction temperatures in Example 13 to 17 were 80°C, 100°C, 120°C, 140°C, and 160°C, respectively.

The heated autoclave was cooled to room temperature, and a reaction product extracted from the autoclave was subjected to solid-liquid separation by filtration. To the filtrate obtained after filtration, tert-butyl alcohol was added as a standard substance, and qualitative and quantitative analysis was performed by GC-MS/FID.

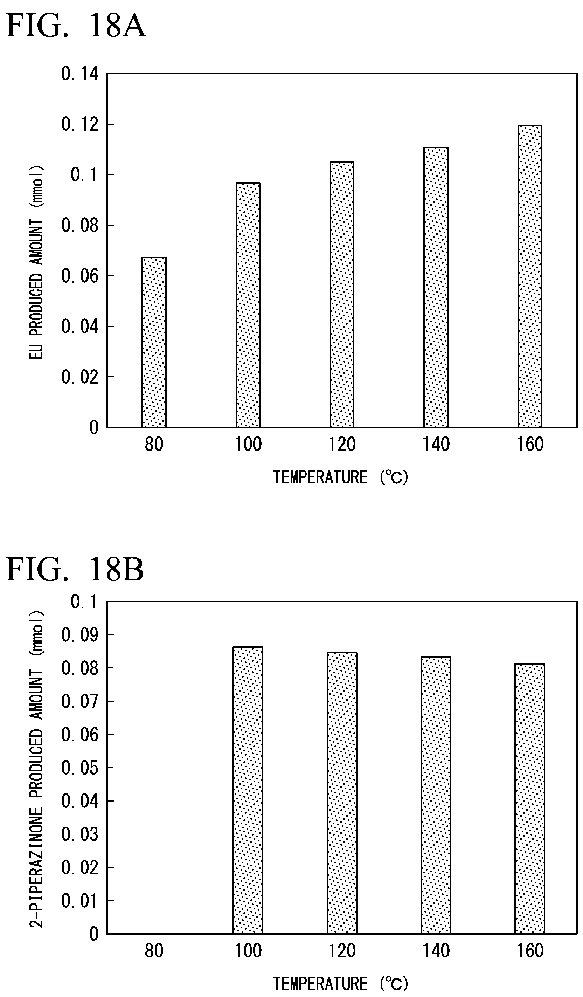

FIG. 18A is a graph illustrating a relationship between a reaction temperature during synthesis of ethylene urea (EU) and a produced amount of ethylene urea in Examples 13 to 17. FIG. 18B is a graph illustrating a relationship between a reaction temperature during synthesis of 2-piperazinone and a produced amount of 2-piperazinone in Examples 13 to 17. A horizontal axis in the graph represents an internal reaction temperature due to the heating of the autoclave, and a vertical axis in the graph represents produced amounts of ethylene urea and 2-piperazinone, respectively. It can be seen from FIG. 18A that the production of ethylene urea was confirmed, and the higher the reaction temperature, the larger the produced amount of ethylene urea. From FIG. 18B, the production of 2-piperazinone can also be confirmed.

### (Comparative Example)

After Ce(OH)₄ was synthesized in the same manner as in Example 11, synthesis of ethylene urea was attempted in the same procedure as in the third step in Examples 13 to 17 without performing CO₂ adsorption on Ce(OH)₄. A reaction temperature by heating an autoclave was 160°C, and a reaction time was 24 hours. However, ethylene urea was not produced.

### Reference Signs List

- 100: gas adsorption device
- 101: tubular electric furnace
- 102: reaction tube
- 103: glass wool
- 104: oxide
- 105: first gas supply unit
- 105A: CO₂ source
- 105B: N₂ source
- 105C: mass flow controller
- 105D: mixer
- 106: second gas supply unit
- 106A: H₂O source
- 106B: pump
- 106C: steam generator
- 107: CO₂ analysis device
- 200: autoclave
- 201: solvent containing ethylenediamine
- 202: titanium zirconium oxide

## Claims

1. A carbon dioxide supply conversion agent to be used for a reaction with an alkylamine compound, the carbon dioxide supply conversion agent comprising:
an oxide; and
carbon dioxide adsorbed on a surface of the oxide.

2. The carbon dioxide supply conversion agent according to claim 1, wherein
the oxide is a titanium zirconium oxide represented by a compositional formula TiₓZr₍₁₋ₓ₎O₂, where 0 < x < 1.

3. The carbon dioxide supply conversion agent according to claim 1, wherein
the oxide is a titanium zirconium oxide represented by a composition formula TiₓZr_{y}Ce_{z}O₂, where 0 < x < 1, 0 < y < 1, and 0 < z < 1.

4. The carbon dioxide supply conversion agent according to claim 1, wherein
the oxide is TiO₂.

5. The carbon dioxide supply conversion agent according to claim 1, wherein
the oxide is ZrO₂.

6. The carbon dioxide supply conversion agent according to claim 1, wherein
the oxide is CeO₂.

7. The carbon dioxide supply conversion agent according to claim 1, wherein
the oxide is a Mg-Al-based layered double hydroxide containing Mg and Al as a constituent metal, and a ratio (Mg/Al) of the number of moles of the Mg contained to the number of moles of the Al contained is 2 or more and 4 or less.

8. The carbon dioxide supply conversion agent according to claim 7, wherein
the Mg-Al-based layered double hydroxide is doped with Ce.

9. The carbon dioxide supply conversion agent according to claim 7, wherein
the Mg-Al-based layered double hydroxide is doped with Zr.

10. The carbon dioxide supply conversion agent according to claim 1, wherein
the oxide is Ce(OH)₄.

11. The carbon dioxide supply conversion agent according to claim 1, wherein
the oxide is Zr(OH)₄.

12. A carbon dioxide utilization method, which is a carbon dioxide utilization method by producing an alkylurea compound using the carbon dioxide supply conversion agent according to any one of claims 1 to 11, the carbon dioxide utilization method comprising:
reacting the carbon dioxide adsorbed on the surface of the oxide with an alkylamine compound represented by the following general formula (1) in the presence of a metal oxide catalyst to produce an alkylurea compound represented by the following general formula (2), 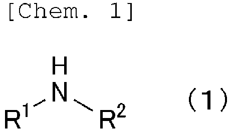 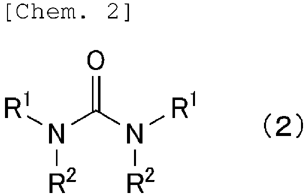
(in the general formula (1) and the general formula (2), R¹ and R² each represent a hydrogen atom, an alkyl group having 1 to 6 carbon atoms, a benzyl group, an aminoalkyl group having 1 to 6 carbon atoms, or a hydroxyalkyl group having 1 to 6 carbon atoms, a combination of R¹ and R² may be any combination except for a combination in which both R¹ and R² are a hydrogen atom, and R¹ in the general formula (1) and R¹ in the general formula (2) are the same, and the same applies to R²).

13. The carbon dioxide utilization method according to claim 12, wherein
the carbon dioxide and the alkylamine compound are reacted in a pressurized state of 0.1 MPa or more and 1.0 MPa or less.

14. The carbon dioxide utilization method according to claim 12, wherein
the carbon dioxide and the alkylamine compound are reacted in a heated state of 0°C or higher and 200°C or lower.
